Europäisches Patentamt

European Patent Office

Office européen des brevets

(11) Publication number: **0 107 119**
**B1**

(12) **EUROPEAN PATENT SPECIFICATION**

(45) Date of publication of patent specification: 04.02.87

(21) Application number: 83109905.6

(22) Date of filing: 04.10.83

(51) Int. Cl.⁴: **A 61 K 47/00,** A 61 K 37/20, A 61 M 1/36

(54) Blood-treating material.

(30) Priority: 04.10.82 JP 173321/82
16.05.83 JP 84197/83

(43) Date of publication of application:
02.05.84 Bulletin 84/18

(45) Publication of the grant of the patent:
04.02.87 Bulletin 87/06

(84) Designated Contracting States:
AT BE CH DE FR GB IT LI NL SE

(56) References cited:
US-A-4 003 792
CHEMICAL ABSTRACTS, vol. 94, 1981, page
135, no. 203541r, Columbus, Ohio, US.
MAITRA, SHYAMAL K. et al.: "Properties of
binding of Escherichia coli endotoxin to
various matrices"
CHEMICAL ABSTRACTS, vol. 94, 1981, page
590, no. 137656d, Columbus, Ohio, US.
RAZIUDDIN, SYED et al.: "Binding of bacterial
endotoxin (LPS) to encephalitogenic myelin
basic protein and modulation of characteristic
biologic activities of LPS"

(73) Proprietor: TORAY INDUSTRIES, INC.
2, Nihonbashi-Muromachi 2-chome Chuo-ku
Tokyo 103 (JP)

(72) Inventor: Kodama, Masashi
91, Minamigasa-cho Kusatsu-shi
Shiga-ken (JP)
Inventor: Katayama, Yutaka
64, Kamigamomidorogaike-cho Kita-ku
Kyoto-shi Kyoto-fu (JP)
Inventor: Tani, Toru
1922-320, Noji-cho
Kusatsu-shi Shiga-ken (JP)
Inventor: Teramoto, Kazuo
25-8, Nango 2-chome
Otsu-shi Shiga-ken (JP)
Inventor: Murakami, Matsuo
880-26, Midori-machi, 1-chome
Omihachiman-shi Shiga-ken (JP)

(74) Representative: Bühling, Gerhard, Dipl.-Chem.
et al
Patentanwaltsbüro Tiedtke-Bühling-Kinne
Grupe-Pellmann-Grams-Struif Bavariaring 4
D-8000 München 2 (DE)

Courier Press, Leamington Spa, England.

(58) References cited:
**O. ZABORSKY: "Immobilized enzymes", 1974, pages 5,9,24-26, CRC Press, Ohio, US CHEMICAL ABSTRACTS, vol. 99, no. 3, July 18, 1983, page 483, no. 20829z, Columbus, Ohio, US. S. LUBINSKY-MINK et al.: "Inter-action of latex-insolubilized endotoxins with murine macrophages: phagocytic responses of endotoxin-responsive (C3HeB/FeJ) and -unresponsive (C3H/HeJ) macrophages in vitro"**

# 0 107 119

**Description**

This invention relates to a blood-treating material having antitumor effect.

The most frequent cause of death in developed countries is cancer. However, powerful carcinostatic agents have not yet been found, unlike antibiotics for infectious diseases.

Lipopolysaccharides derived from cell walls of Gram-negative bacteria are substances called "endotoxins" or "pyrogens". It has been known that the lipopolysaccharides have harmful effects such as pyrogenic effects, Schwartzman phenomenon and lethal toxicity. It is also known that they have antitumor effects against malignant tumors.

Substances having anti-tumor effects are desirable, since specific remedies for malignant tumors have not yet been developed. However, the lipopolysaccharides cannot be used safely, since their fatal doses are very close to the minimum effective doses in the treatment of the tumors.

From the Journal of Clinical Microbiology, vol. 13(1), pages 49 to 53, 1981 (Chemical Abstracts, vol. 94, No. 20 35 41r, 1981), it is known to immobilize lipopolysaccharides by using different insoluble carriers. The carriers mentioned are ion exchange resins such as Dowex® 1-X2, polystyrene and activated charcoal which absorb lipopolysaccharide from an aqueous solution. An ionic binding or affinity interactions between groups of the insoluble carrier and groups of the lipopolysaccharide cause the binding between the insoluble carrier and the lipopolysaccharide. It can be gathered from the page 51, Table I and from the page 52, left-hand column, lines 5 to 8 of the last paragraph that the endotoxin-bound polystyrene was incubated at 37°C for 1 h with serum, but only 85% of the endotoxin was still bound to the polystyrene, so that about 15% of the endotoxin was released. Also in the case of the endotoxin bound to an anion exchange resin, it is to be expected that the endotoxin is released, since the anion exchange resin absorbed 42% of endotoxin in serum, but 100% of endotoxin in salt solution. Accordingly the lipopolysaccharide is bound reversibly and cannot safely be used for the treatment of blood. Furthermore, the amount of lipopolysaccharide absorbed is very small and amounted to only about 15 µg of lipopolysaccharide per gramme of the insoluble carrier (see page 50, left column, lines 41 to 48).

From the Journal of Immunology, vol. 126(3), pages 1030 to 1035, 1981 (Chemical Abstracts vol. 94, No. 137656 d, 1981) the binding of lipopolysaccharide to encephalitogenic myelin basic protein is known. This protein is isolated from the central nervous system. The binding of the endotoxin with the protein results in generation of lower molecular weight aggregates with decreased isopycnic density. This literature does not disclose an insoluble carrier.

Object of the present invention is to provide a blood treating material wherein a high amount of lipopolysaccharide can be fixed and which can safely be used.

The present invention provides a blood-treating material comprising a lipopolysaccharide derived from cell walls of Gram-negative bacteria and bonded by condensation to an insoluble carrier containing a primary and/or secondary amino group or a carboxyl group.

Further the invention provides a process for producing the blood-treating material characterized by reacting a condensing agent with a mixture of the lipopolysaccharide and an insoluble carrier, having at least one group selected from a primary or secondary amino group or a carboxyl group.

The present invention makes it possible to reduce the lethal toxicity of the above-mentioned lipopolysaccharides and provide a blood-treating material that can be used safely for therapy of cancer, because this blood-treating material has antitumor effects similar to those of the lipopolysaccharides derived from cell walls of Gram-negative bacteria but has no fatal effects.

The lipopolysaccharides derived from cell walls of Gram-negative bacteria herein are lipid-polysaccharide complexes or lipid-polysaccharide-protein complexes localized in the outer layers of the cell walls of Gram-negative bacteria including Gram-negative cocci such as *Neisseria gonorrhoeae*, Gram-negative aerobic bacilli such as *Pseudomonas aeruginosa, Brucella abortus* and *Bordetella pertussis* and Gram-negative facultative anaerobic bacilli such as *Escherichia coli, Salmonella typhi, Shigella dysenteriae, Klebsiella pneumoniae, Serratia marcescens, Proteus vulgaris, Yersinia enterocolitica* and *Vibrio cholerae*. These lipopolysaccharides can be extracted from the Gram-negative bacteria by known processes. As typical processes, there may be mentioned the phenol-water extraction process (Otto Westphal et al., Z. Naturforsch., 7B: 148—155(1952)), trichloroacetic acid extraction (A. Boivin and L. Meserobeanu, Comp. Rend. Soc. Biol., *128*, 5 (1938)), butanol extraction (D. C. Morrison and L. Leive, J. Biol. Chem. *250*, (3) 2911 (1975)) and ethylenediamine-tetraacetic acid-water extraction (L. Leive et al., J. Biol. Chem., *243*, 6384 (1968)). From the viewpoint of easiness of the immobilization it is preferred that the lipopolysaccharides have a protein content of 0.1 to 50%, particularly 1 to 20%. The molecular weights of the lipopolysaccharides vary, depending on the measuring method and conditions generally, in the range of several thousands to several millions, since their molecules are liable to associate with each other.

The insoluble carriers herein involve substantially insoluble carriers having a primary and/or secondary amino group, or a carboxyl group and release no toxic substance under the blood-treating conditions.

The insoluble carriers consist of aromatic polymeric material containing a primary and/or secondary amino group or a carboxyl group as the substituent on the aromatic nucleus. Suitable carriers used in the present invention include, for example, (1) polystyrene or styrene/divinylbenzene copolymer in which a primary and/or secondary amino group, or carboxyl group has been introduced as a substituent into the

3

aromatic nucleus, (2) the same carrier as in the above item (1) but containing methylene-crosslinked or sulfone-cross-linked polystyrene used in place of the polystyrene, (3) acrylic acid/acrylonitrile copolymer, (4) acrylic acid/styrene copolymer, (5) nylon 6, (6) nylon 6.6, (7) polyethylene terephthalate, (8) carboxyl group-containing insolubilized cellulose and (9) aminoethylated cellulose. Among them, carriers containing a vinyl polymer as the backbone polymer and particularly those containing a styrene polymer as the backbone polymer are preferred, since they have high acid and alkali resistances.

The form of the insoluble carriers is not particularly limited provided that they have an adequate surface area and a sufficient mechanical strength to withstand external forces during their use. Generally, they are used in the form of granules, fibres, films, hollow filaments and membranes. The fiber is especially an island-in-sea type composite fiber. The fiber is used in form of non-woven fabric, paper, woven fabric or knitted fabric.

According to the present invention, the lipopolysaccharide may be immobilized on the insoluble carrier by any process wherein the condensation reactivity of the primary and/or secondary amino or carboxyl group of the insoluble carrier is utilized. In the process according to the invention (1) a condensing agent for peptide synthesis is added to a mixture of the insoluble carrier and an aqueous solution of the lipopolysaccharide, or (2) a carboxyl group-containing insoluble carrier is treated with N-hydroxysuccinimide N-hydroxy-5-norbonene-endo-2,3-dicarboximide or 1-hydroxybenzotriazole. Then the condensing agent for peptide synthesis and then the carrier is mixed with the lipopolysaccharide solution.

As the condensing agent for the peptide synthesis, there may be mentioned 1-ethyl-3-(3-dimethylaminopropyl) carbodiimide, 1-cyclohexyl-3-(2-morpholinoethyl)carbodiimide 1-cyclohexyl-3-(2-morpholinoethyl)-carbodiimide metho-p-toluenesulfonate, N,N'-dicyclohexylcarbodiimide and Woodward reagent K.

As for the density of the lipopolysaccharide immobilized on the insoluble carrier in the present invention, it is desirable to immobilize at least 10 μg, more preferably 1 mg of the lipopolysaccharide per square meter of the surface area of the carrier, since if the immobilization density is too low, a large amount of the carrier is required. Accordingly, when the insoluble carrier has a surface area of 0.01 to 100 m$^2$/g, it is preferred that at least 0.1 milliequivalents/g of the primary and/or secondary amino group or carboxyl group is contained therein.

The blood-treating material of the present invention may be used in such a way that it is charged into an extracorporeal circulation column and contacted with the whole blood or blood plasma continuously or intermittently or, alternatively, the blood-treating material is charged into an injector, then the whole blood or plasma is sucked therein and the resulting liquid is returned into the body.

The following examples further illustrate the present invention.

Example 1
Preparation of insoluble carrier

50 g of an islands-in-sea type composite fiber (having 16 islands, single fiber titre of 2.6 den, tensile strength of 2.9 g/den, elongation of 50%) comprising 50 parts of a polypropylene (Mitsui "Noprene"® J3HG) as an island component and a mixture of 46 parts of a polystyrene ("Styron"® 666) and 4 parts of a polypropylene (Sumitomo "Noprene"® WF-727-F) as an island component were immersed in a mixed solution of 50 g of N-hydroxymethyl-α-chloroacetamide, 400 g of nitrobenzene, 400 g of 98% sulfuric acid and 0.85 g of paraformaldehyde and the reaction is carried at 20°C for 1 h. The fiber was removed from the reaction liquid and placed into 5 l of ice/water at 0°C to terminate the reaction. The fiber was washed with water and then nitrobenzene was removed from the fiber by extraction with methanol. The fiber was dried at 50°C in vacuum to obtain 71 g of chloroacetamidomethylated fiber. Then, 40 g of the fiber were immersed in ethylenediamine at 15°C and a reaction was carried out at 15 to 20°C for 24 h to obtain ethylenediaminoacetamidomethylated fiber. The fiber containing 1.8 mmol/g of an ethylenediamino group had primary and secondary amino groups but did not have tertiary or quaternary amino groups. The fiber had a water content of 1.0 g per gram (1.0 g) of the dry fiber at pH 7.4. Under this condition, the single yarn diameter was 40 to 44 μm.

Preparation of the blood treating material

32 g of the above-mentioned amino group-containing polystyrene fiber were swollen by immersing the same in 300 ml of water for a whole day and night. The fiber was then mixed with 500 ml of a 0.4 mg/l aqueous solution of a lipopolysaccharide obtained from *Escherichia coli* 055:B5 (a product of Difco Laboratories Co.; obtained by extraction with trichloroacetic acid; the protein content is 10%). Then, 5.0 g of 1-ethyl-3-(3-dimethylaminopropyl)carbodiimide were added thereto in portions while the pH of the mixture was maintained at 4.5 to 6.0 with 1N hydrochloric acid and 1N sodium hydroxide. A solution thus obtained was shaken at room temperature for 2 days. The fiber was taken out and washed with 1 l of water three times. The fiber was then immersed in 1 l of boiling water for 30 min. This immersion was repeated three times. The fiber was washed with a 0.07M phosphate buffer solution (pH 7.4) till the pH of the wash solution was 7.4 to obtain the fiber on which the lipopolysaccharide was immobilized.

The concentrations of the lipopolysaccharide in the immobilization mother liquor and the wash solution were determined by phenol/sulfuric acid method (wherein 5 ml of concentrated sulfuric acid was added to a mixture of 1 ml of the sample and 1 ml of a 5% aqueous phenol solution and the absorbance of

4

the mixture at 485 nm was measured). The amount of immobilized lipopolysaccharide was estimated from that of lipopolysaccharide remaining in the mother solution.

The amount of the immobilized lipopolysaccharide was 5.0 mg/g.

0.6 g of the above-mentioned lipopolysaccharide-immobilized fiber were washed with 200 ml of isotonic sodium chloride solution according to the Japanese Pharmacopoeia and then immersed in 50 ml of the same solution. The resulting solution was heated to 38°C for 3 h. Thereafter, the amount of the lipopolysaccharide in the isotonic sodium chloride solution was determined by the Limulus-Pregel method. The amount was below 1 ng/ml.

A biological test of the blood-treating material Fibrosarcoma MC-BL was induced in BALB/C mice by inoculation with methylcholanthrene. Five hundred thousand tumor cells were taken and subcutaneously injected into the backs of BALB/C mice to obtain tumor-bearing mice. The tumor grew into a size of 2.1 mm diameter 5 days after the inoculation, 6.3 mm 10 days thereafter and 8.9 mm 12 days thereafter.

0.6 g of the above-mentioned lipopolysaccharide-immobilized fiber (containing 1.0 mg of the lipopolysaccharide) prepared in Example 1 were immersed in 20 ml of human plasma at 37°C for 180 min. The plasma was given to the tumor-bearing mice by intravenous injection 11 days after the inoculation (tumor diameter: 7 mm), 13 days and 15 days after the inoculation in an amount of 0.5 ml each time. In one mouse out of a total of 10 mice, the tumor was completely necrotized and disappeared. In the balance (9 mice), a remarkable softening of the tumor was recognized. The same experiments as above were carried out using 10 ml of human plasma treated at 56°C for 30 min as a control. In the latter case, necrosis, softening or bleeding of the tumor was not observed at all.

Example 2

0.4 g of the lipopolysaccharide-immobilized fiber (2.0 mg of lipopolysaccharide) prepared in Example 1 were immersed in 40 ml of plasma obtained from 100 BALB/C mice at 37°C for 180 min. The plasma was given to the tumor-bearing mice obtained in the same way as in Example 1 by intravenous injection 11, 13 and 15 days after the inoculation in an amount of 1.0 ml each time. In one mouse out of a total of 10 mice, the tumor disappeared completely. In the rest (9 mice), a remarkable softening of the tumor was recognized.

Example 3

0.2 g of the lipopolysaccharide-immobilized fiber prepared in Example 1 were immersed in 20 ml of human plasma at 37°C for 180 min. 0.5 ml portions of the plasma were mixed with 2.5 ml portions of human plasma. The mixture was given to the tumor-bearing mice obtained in the same way as in Example 1 by intraperitoneal injection 11, 13, 15, 17 and 19 days after the inoculation. The tumor disappeared completely in 2 mice out of a total of 7 mice.

Example 4

Tumor-bearing rabbits were obtained by intradermal injection of $5 \times 10^5$ VX2 tumor cells (suspended in 0.3 ml of phosphate buffered saline containing 10% fetal calf serum) into the backs of New Zealand White rabbits (male).

Blood-treating columns were prepared by filling 0.8 g of bundled blood-treating material or carrier obtained in the same way as in Example 1.

Tumor-bearing rabbits underwent extracorporeal perfusion through the above mentioned columns at a flow rate of 15 ml/min for 90 minutes via cannulation of the carotid artery and the jugular vein under anesthesia (with sodium pentobarbital intraperitoneal injection) 5 days after inoculation. Heparin (500 U/kg-bodyweight of rabbit) was added into the circuit at the beginning of the perfusion. Thereafter, the weight of the rabbits and the size of the tumor were examined. Nine tumor-bearing rabbits (group 1) were used for the blood-treating material and five (group 2) were used for the carrier. Six tumor-bearing rabbits (group 3) were observed without extracorporeal perfusion.

The size of the tumor was calculated by the equation: the diameter of the tumor$=(ab)^{1/2}$ where a and b are the longest diameter of the tumor and the diameter taken rectangular to the longest diameter, respectively.

Changes of tumor-size and weight of rabbits were shown in Table I and II respectively.

From these results, it is indicated that the bloodtreating material of the present invention is effective for increasing the weight of the tumor-bearing rabbit as well as for decreasing the tumor size.

TABLE I
Average diameter of the tumors (mm)

| Group of rabbits | Time after inoculation (day) | | | | | | |
|---|---|---|---|---|---|---|---|
| | 5 | 10 | 15 | 20 | 25 | 30 | 35 |
| 1 | 9.3 | 10.5 | 11.0 | 12.5 | 9.2 | 7.5 | 7.6 |
| 2 | 9.2 | 11.0 | 14.8 | 18.2 | 23.1 | 40.3 | 55.5 |
| 3 | 9.0 | 13.5 | 17.6 | 20.3 | 25.3 | 40.9 | 57.1 |

TABLE II
Average weight of rabbits (kg)
Time after inoculation (day)

| Group of rabbits | 0 | 5 | 10 | 15 | 20 | 25 | 30 | 35 | 40 | 45 | 50 |
|---|---|---|---|---|---|---|---|---|---|---|---|
| 1 | 2.17 | 2.15 | 2.06 | 2.10 | 2.23 | 2.34 | 2.45 | 2.51 | 2.60 | 2.72 | 2.79 |
| 2 | 2.25 | 2.23 | 2.10 | 2.10 | 2.11 | 2.10 | 2.06 | 2.03 | 1.94 | 1.89 | 1.82 |
| 3 | 2.27 | 2.25 | 2.22 | 2.24 | 2.32 | 2.38 | 2.35 | 2.40 | 2.23 | 2.07 | 1.90 |

**Claims**

1. A blood-treating material comprising a lipopolysaccharide derived from cell walls of Gram-negative bacteria and bonded by condensation to an insoluble carrier containing a primary and/or secondary amino group or a carboxyl group.

2. A blood-treating material according to Claim 1, wherein the content of the primary and/or secondary amino group or the carboxyl group is at least about 0.1 meq/g of the insoluble carrier.

3. A blood-treating material according to Claim 1, wherein the insoluble carrier consists of aromatic polymeric material containing a primary and/or secondary amino group or a carboxyl group as the substituent on the aromatic nucleus.

4. A blood-treating material according to Claim 1, wherein said material is in the form of a fiber or a film.

5. A blood-treating material according to Claim 4, wherein said fiber is an islands-in-sea type composite fiber.

6. A blood-treating material according to Claim 4, wherein said fiber is in the form of non-woven fabric, paper, woven fabric or knitted fabric.

7. The blood-treating material according to Claim 1, wherein the amount of the immobilized lipopolysaccharide derived from cell walls of Gram-negative bacteria is at least 1 mg per square meter of the surface area of the blood-treating material.

8. A process for preparing a blood-treating material characterized by reacting a condensing agent with a mixture of lipopolysaccharide and an insoluble carrier having at least one group selected from a primary or secondary amino group or a carboxyl group.

9. The process according to Claim 8, wherein said condensing agent is at least one compound selected from 1-ethyl-3-(3-dimethylaminopropyl)-carbodiimid, 1-cyclohexyl-3-(2-morpholinoethyl)-carbodiimide, 1-cyclohexyl-3-(2-morpholinoethyl)-carbodiimide metho-p-toluenesulfonate, N,N'-dicyclohexylcarbodiimide and Woodward Reagent K.

10. The process according to Claim 8, wherein said insoluble carrier having carboxyl group is activated by at least one compound selected from N-hydroxysuccinimide, N-hydroxy-5-norbornene-endo-2,3-dicarboximide and 1-hydroxybenzotriazole.

**Patentansprüche**

1. Substanz zur Blutbehandlung, die ein Lipopolysaccharid enthält, das aus Zellwänden gramnegativer Bakterien erhalten worden und durch Kondensation an einen unlöslichen Träger, der eine primäre und/oder eine sekundäre Aminogruppe oder eine Carboxylgruppe enthält, gebunden worden ist.

2. Substanz zur Blutbehandlung nach Anspruch 1, bei der der Gehalt der primären und/oder der sekundären Aminogruppe oder der Carboxylgruppe mindestens etwa 0,1 mval/g des unlöslichen Trägers beträgt.

3. Substanz zur Blutbehandlung nach Anspruch 1, bei der der unlösliche Träger aus einer aromatischen polymeren Substanz besteht, die als Substituent an dem aromatischen Kern eine primäre und/oder eine sekundäre Aminogruppe oder eine Carboxylgruppe enthält.

4. Substanz zur Blutbehandlung nach Anspruch 1, bei der die erwähnte Substanz die Form von Fasern oder einer Folie hat.

5. Substanz zur Blutbehandlung nach Anspruch 4, bei der die erwähnten Fasern Verbundfasern des "Inseln-im-Meer"-Typs sind.

6. Substanz zur Blutbehandlung nach Anspruch 4, bei der die erwähnten Fasern die Form von Faservlies, Papier, gewebtem Stoff oder Maschenware haben.

7. Substanz zur Blutbehandlung nach Anspruch 1, bei der die Menge des aus Zellwänden gramnegativer Bakterien erhaltenen, immobilisierten Lipopolysaccharids mindestens 1 mg je Quadratmeter der Oberfläche der Substanz zur Blutbehandlung beträgt.

8. Verfahren für die Herstellung einer Substanz zur Blutbehandlung, dadurch gekennzeichnet, daß ein Kondensationsmittel mit einer Mischung aus Lipopolysaccharid und einem unlöslichen Träger, der mindestens eine Gruppe hat, die aus einer primären oder einer sekundären Aminogruppe oder einer Carboxylgruppe ausgewählt ist, zur Reaktion gebracht wird.

9. Verfahren nach Anspruch 8, bei dem das erwähnte Kondensationsmittel mindestens eine Verbindung ist, die aus 1-Ethyl-3-(3-dimethylaminopropyl)-carbodiimid, 1-Cyclohexyl-3-(2-morpholino-ethyl)-carbodiimid, 1-Cyclohexyl-3-(2-morpholinoethyl)-carbodiimidmetho-p-toluolsulfonat, N,N'-Dicyclohexylcarbodiimid und Woodward-Reagens K ausgewählt ist.

10. Verfahren nach Anspruch 8, bei dem der erwähnte unlösliche Träger, der eine Carboxylgruppe hat, durch mindestens eine Verbindung, die aus N-Hydroxysuccinimid, N-Hydroxy-5-nobornen-endo-2,3-dicarboximid und 1-Hydroxybenzotriazol ausgewählt ist, aktiviert ist.

**Revendications**

1. Matériau pour le traitement du sang comprenant un lipopolysaccharide provenant des parois de cellules de bactéries Gram- et lié par condensation à un porteur insoluble contenant un groupe amino primaire et/ou secondaire ou un groupe carboxyle.

2. Matériau de traitement du sang selon la revendication 1, dans lequel la teneur en groupe amino primaire et/ou secondaire ou en groupe carboxyle est au moins environ 0,1 meq/g du porteur insoluble.

3. Matériau de traitement du sang selon la revendication 1, dans lequel le porteur insoluble est constitué d'un matériau polymère aromatique contenant un groupe amino primaire et/ou secondaire ou un groupe carboxyle comme substituant sur le noyau aromatique.

4. Matériau de traitement du sang selon la revendication 1, dans lequel ce matériau a la forme d'une fibre ou d'une pellicule.

5. Matériau de traitement du sang selon la revendication 4, dans lequel la fibre est une fibre composite du type îles dans la mer.

6. Matériau de traitement du sang selon la revendication 4, dans lequel la fibre a la forme d'un tissu no tissé, de papier, de tissu tissé ou de tissu tricoté.

7. Matériau de traitement du sang selon la revendication 1, dans lequel la quantité de lipopolysaccharide immobilisé provenant des parois des cellules de bactéries Gram- est au moins 1 mg par mètre carré de l'aire du matériau de traitement du sang.

8. Procédé de préparation d'un matériau de traitement du sang, caractérisé en ce qu'il consiste à faire réagir un agent de condensation avec un mélange de lipopolysaccharide et d'un porteur insoluble ayant au moins un groupe choisi parmi un groupe amino primaire ou secondaire ou un groupe carboxyle.

9. Procédé selon la revendication 8, dans lequel l'agent de condensation est au moins un composé choisi parmi les composés suivants: 1-éthyl-3-(3-diméthylaminopropyl)-carbodiimide, 1-cyclo-hexyl-3-(2-morpholinoéthyl)-carbodiimide, 1-cyclohexyl-3-(2-morpholinoéthyl)-carbodiimide, métho-p-toluènesulfonate, N,N'-dicyclohexylcarbodiimide et Réactif Woodward K.

10. Procédé selon la revendication 8, dans lequel le porteur insoluble comportant un groupe carboxyle est activé par au moins un composé choisi parmi les composés suivants: N-hydroxysuccinimide, N-hydroxy-5-norbornène-endo-2,3-dicarboximide et 1-hydroxybenzotriazole.